(19) Europäisches Patentamt
European Patent Office
Office européen des brevets

(11) **EP 2 692 438 A1**

(12) **EUROPEAN PATENT APPLICATION**

(43) Date of publication:
**05.02.2014 Bulletin 2014/06**

(21) Application number: **12005628.8**

(22) Date of filing: **02.08.2012**

(51) Int Cl.:
*B01J 29/87* *(2006.01)*  *B01J 29/44* *(2006.01)*
*C10G 35/06* *(2006.01)*  *C10G 35/095* *(2006.01)*
*C07C 2/76* *(2006.01)*  *C07C 15/02* *(2006.01)*

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Saudi Basic Industries Corporation
Riyadh 11422 (SA)**

(72) Inventor: **The designation of the inventor has not
yet been filed**

(74) Representative: **Vroemen, Maurice
SABIC Petrochemicals B.V.
Intellectual Property Group
P.O. Box 3008
6160 GA Geleen (NL)**

(54) **Catalyst composition for the production of aromatic hydrocarbons**

(57) The invention relates to a catalyst composition suitable for conversion of alkanes having 3 to 12 carbon atoms per molecule to aromatic hydrocarbons, wherein the catalyst composition comprises $M_N/M_A$/Ga-zeolite, wherein $M_N$ stands for noble metals and $M_A$ stands for alkali metals and/or alkaline earth metals, and wherein $M_N/M_A$/Ga-zeolite is a zeolite comprising 0.01 -10 wt % of $M_N$ with respect to the total $M_N/M_A$/Ga-zeolite, 0.01 - 10 wt % of $M_A$ with respect to the total $M_N/M_A$/Ga-zeolite and 0.01 - 10 wt % Ga with respect to the total $M_N/M_A$/Ga- zeolite.

The invention also relates to a process for the preparation of said catalyst composition and to a process for the production of aromatic hydrocarbons comprising the step of contacting a feedstream comprising an alkane selected from the group of alkanes having from 3 to 12 carbon atoms per molecule and any mixtures of alkanes having from 3 to 12 carbon atoms per molecule with said catalyst composition to form aromatic hydrocarbons.

EP 2 692 438 A1

**Description**

The invention relates to a catalyst composition comprising a zeolite, a process for the production of the catalyst composition, a process for the production of aromatic hydrocarbons using the catalyst composition and to the use of the catalyst composition.

The aromatization of alkanes having 3 to 12 carbon atoms to yield mixtures of benzene, toluene and xylenes (commonly known as BTX) has been the subject of study for many years. BTX are important building blocks in the petrochemical industry and are also utilized as a booster to enhance the octane number in gasoline.

Traditionally, BTX products are produced by the catalytic reforming of alkanes having for example 6 to 12 carbon atoms, commonly referred to as petroleum naphtha. Recently there has been considerable effort invested in developing catalyst compositions that attempt to combine a good degree of conversion of alkanes to the hydrocarbons with selectivity for certain aromatic hydrocarbons, for example benzene. The selectivity for aromatic hydrocarbons is also referred to herein as BTX selectivity.

A need that has yet to be met by the catalyst compositions disclosed in the prior art is a catalyst composition that provides both a high conversion of alkanes having 3 to 12 carbon atoms, preferably for light naphtha, which are alkanes having 6-8 carbon atoms and a high selectivity for aromatic hydrocarbons, in particular for benzene.

A ZSM-5 catalyst comprising gallium used in the aromatization of hydrocarbons is disclosed in CN1296861. The catalyst composition comprises ZSM-5 zeolite, Ga and one metal chosen from the group consisting of La, Ag, Pd, Zn and Re. In a preferred embodiment the composition comprises 63-99 wt % ZSM-5, 0.8-1.6 wt% Ga, 0.1-1.0 wt% of the metal selected from the group consisting of La. Ag, Pd, Zn and Re. Although compositions according to the invention of CN1296861 gave a high conversion in the range of 94-100%, the benzene selectivity was low, for example in the range of 38-52%, after 30 hours.

EP0283212 discloses a process for producing aromatic hydrocarbon compounds comprising 2 to 6 carbons with a catalyst composition comprising gallium and one lanthanide element. The composition may comprise 0.2-1 % w/w of gallium and from 0.1 to 0.8 % w/w of lanthanum. The catalyst composition has a benzene selectivity of approximately 56% after 24 hours.

US7164052 discloses that aromatic hydrocarbon compounds are produced by a process of contacting one or more aliphatic hydrocarbons containing from 3 to 6 carbon atoms with a catalytic composition comprising (i) gallium, (ii) at least one lanthanide element and (iii) a zeolite of the MIF family. The obtained wt % of BTX compounds ranged from 18 % to 60 %, but no selectivity for benzene was reported.

US5006497A discloses that a single shape selective zeolite e.g. ZSM-5 with a controlled amount of an aromatization component such as gallium, may promote both paraffin cracking / isomerization and aromatization. The conversion to aromatic hydrocarbons is, however, very low (for example 18.5 %).

WO2008/080517 discloses a process wherein aromatic hydrocarbons are produced by contacting alkanes having 1 to 6 carbon atoms with a catalyst composition comprising a zeolite modified with gallium and lanthanum. The gallium is present in an amount of at most 0.95 wt% with respect to the total of zeolite and gallium. The process was operated at 580 °C and yielded a conversion of propane of at most 85 %. The selectivity of benzene, toluene or xylene was only 51 wt % (see Table 4 of WO2008/080517).

WO2005/085157A1 discloses a process for the aromatization of hydrocarbons comprising: a) contacting an alkane containing 2 to 6 carbon atoms per molecule with at least one catalyst containing a gallium zeolite on which platinum has been deposited; and b) recovering the aromatic product. In the examples, the BTX selectivity from propane is only 50 wt%. Further, the BTX selectivity decreases with time on stream (TOS).

It is the object of the current invention to provide a catalyst composition that is able to convert alkanes to aromatic hydrocarbons, with a high conversion and with a high selectivity for aromatic hydrocarbons, preferably for benzene.

The object of the invention is achieved by a catalyst composition suitable for conversion of alkanes having 3 to 12 carbon atoms per molecule to the aromatic hydrocarbons, wherein the catalyst composition comprises $M_N/M_A$/Ga-zeolite, wherein $M_N$ stands for one or more noble metals and $M_A$ stands for one or more alkali metals and/or alkaline earth metals.

Preferably, $M_N/M_A$/Ga-zeolite is a zeolite comprising 0.01 -10 wt % of $M_N$ with respect to the total $M_N/M_A$/Ga-zeolite, 0.01 — 10 wt % of $M_A$ with respect to the total $M_N/M_A$/Ga-zeolite and 0.01 — 10 wt % Ga with respect to the total $M_N/M_A$/Ga-zeolite.

The inventors found that a composition according to the invention enabled a high conversion of an alkane, in particular of an alkane having 6 to 8 carbon atoms per molecule (light naphtha) to an aromatic hydrocarbon with values as high as, for example, 70-100 % and could be combined with a high benzene selectivity of, for example, 70-80 %. An additional advantage of the catalyst composition disclosed herein may be that the catalyst composition maintains its activity over longer periods of time.

In the framework of the invention, with alkane is meant a hydrocarbon of formula $C_2H_{2n+2}$. For example, the

alkane can have from 3 to 12, for example from 4 to 10, preferably from 6 to 8 carbon atoms per molecule. For example, the alkane may be butane, pentane hexane, heptane, octane, nonane, decane or a mixture thereof. Preferably, the alkane is chosen from the group of hexane, heptane, octane and mixtures thereof.

It is to be understood that also isomers of the alkanes are included by the term 'alkane'. For example, in case the alkane is hexane, the alkane may be n-hexane; 2-methylpentane; 3-methylpentane; 2,3-dimethylbutane; 2,2-dimethylbutane or any mixture thereof. For example, in case the alkane is heptane, the alkane may be n-heptane; 2-methylhexane; 3-methylhexane; 2,2-dimethylpentane; 2,3-dimethylpentane; 2,4-dimethylpentane; 3,3-dimethylpentane; 3-ethylpentane; 2,2,3-trimethylbutane or any mixture thereof For example, in case the alkane is octane, the alkane may be n-octane; 2-methylheptane; 3-methylheptane; 4-methylheptane; 3-ethylhexane; 2,2-dimethylhexane; 2,3-dimethylhexane; 2,4-dimethylhexane; 2,5-dimethylhexane; 3,3-dimethylhexane; 3,4-dimethylhexane; 3-ethyl-2-methylpentane; 3-ethyl-3-methylpentane; 2,2,3-trimethylpentane; 2,2,4-trimethylpentane; 2,3,3-trimethylpentane; 2,3,4-trimethylpentane; 2,2,3,-tetramethylbutane or any mixture thereof.

Preferably, the alkane is chosen from the group of n-hexane, n-heptane, n-octane and mixtures thereof. However, it is also possible to use a mixture of isomers of any chosen alkane, for instance a mixture of isomers of hexane, heptane or octane. For instance a mixture of isomers of hexane in which the amount of n-hexane is for example at least 95% by weight, for example at least 97% by weight or for example at least 99% by weight based on the total amount of hexane.

In this application $M_N$ is used as an abbreviation for noble metals. $M_A$ is used as an abbreviation for alkali metal and/or alkaline earth metals. For the avoidance of doubt, in this application the composition according to the invention described as $M_N/M_A$/Ga-zeolite is therefore understood to comprise of a zeolite comprising gallium, one ore more alkali metals and/or alkaline earth metals and one or more noble metals.

The zeolite used in the process according to the invention can comprise crystalline or amorphous zeolite structures with crystalline materials being preferred, because of their more homogeneous pore size and channeling framework structures.

As used herein, the term "zeolite" or "aluminosilicate zeolite" relates to an aluminosilicate molecular sieve. These inorganic porous materials are well known to the skilled person. An overview of their characteristics is for example provided by the chapter on Molecular Sieves in Kirk-Othmer Encyclopedia of Chemical Technology, Volume 16, p 811-853; in Atlas of Zeolite Framework Types, 5th edition, (Elsevier, 2001).

Aluminosilicate zeolites are generally characterized by the Si/Al ratio of the framework. This ratio may vary widely in the catalyst composition used in the process according to the invention. Preferably, the Si/Al ratio is from about 5 to 1000, preferably from about 8 to 500 or preferably from 10 to 100 or more preferably from 10 to 200. Any aluminosilicate that shows activity in converting alkanes to aromatic hydrocarbons, before modifying it with a specific metal, may be applied. Examples of suitable materials include the mordenite framework inverted (MFI) and other zeolite structures known to the skilled person, for example MEL, MWW, BEA, MOR, LTL and MTT type. Preferred materials are those known as ZSM-5, ZSM-11, ZSM-8, ZSM-12, ZSM-22, ZSM-23, ZSM-35, ZSM-38, and beta aluminosilicates. Most preferably the zeolite is a MFI type zeolite, for example a ZSM-5 zeolite.

The term "medium pore zeolite" is commonly used in the field of zeolite catalyst compositions. Accordingly, a medium pore size zeolite is a zeolite having a pore size of 5-6 Å. Suitable medium pore size zeolites are 10-ring zeolites. i.e. the pore is formed by a ring consisting of 10 $SiO_4$ tetrahedra. Zeolites of the 8-ring structure type are called small pore size zeolites; and those of the 12-ring structure type, like for example beta zeolite, are also referred to as large pore sized. In the above cited Atlas of Zeolite Framework Types, various zeolites are listed based on ring structure. Preferably, the zeolite is a medium pore size aluminosilicate zeolite.

The zeolite of the present invention may be dealuminated. Preferably, the silica ($SiO_2$) to alumina ($Al_2O_3$) molar ratio of the ZSM-5 zeolite is in the range of 10 to 200. Means and methods to obtain dealuminated zeolite are well known in the art and include, but are not limited to the acid leaching technique; see e.g. Post-synthesis Modification I; Molecular Sieves, Volume 3; Eds. H. G. Karge, J. Weitkamp; Year (2002); Pages 204-255.

It is preferred that the zeolite is in the hydrogen form: i.e. having at least a portion of the original cations associated therewith replaced by hydrogen. Methods to convert an aluminosilicate zeolite to the hydrogen form are well known in the art. A first method involves direct ion exchange employing an acid. A second method involves base-exchange using ammonium salts followed by calcination.

The catalyst composition of the invention comprises one or more noble metals ($M_N$). The noble metal may be, for example, platinum (Pt), palladium (Pd), iridium (Ir), rhodium (Rh) and ruthenium (Ru) and mixtures thereof. Preferably the noble metal is platinum (Pt). Accordingly, the catalyst composition provided by the present invention comprises preferably for example at least 0.01 wt %, for example at least 0.03 wt %, for example at least 0.05 wt %, for example at least 1.0 wt % noble metal with respect to the total $M_N/M_A$/Ga-zeolite and / or for example at most 0.05 wt %, for example at most 0.5 wt %, for example at most 1.0 wt %, for example at most 10 wt % noble metal with respect to the total $M_N/M_A$/Ga-zeolite. Preferably the catalyst composition comprises for example 0.01 — 10 wt%, for example 0.02 — 5.0 wt % noble metal with respect to the total $M_N/M_A$/Ga-zeolite.

Furthermore, the catalyst composition comprises one or more alkali metals and/or alkaline earth metals. The alkali metal and/or alkaline earth metal may be chosen from the group of sodium (Na), lithium (Li), potassium (K), rubidium (Rb), cesium (Cs), magnesium (Mg), calcium (Ca), strontium (Sr) and barium (Ba) and mixtures thereof. Preferably the alkali metal and/or alkaline earth metal is cesium. The alkali metal and/or alkaline earth metal is present in the composition in for example at least 0.01 wt %, for example at least 0.03 wt %, for example at least 0.05 wt, % for example at least 1.0 wt % alkali metal and/or alkaline earth metal with respect to the total $M_N/M_A$/Ga-zeolite and/or for example at most 0.05 wt %, for example at most 0.5 wt %, for example at most 1.0 wt%, for example at most 10 wt % alkali metal and/or alkaline earth metal ($M_A$) with respect to the total $M_N/M_A$/Ga-zeolite. Preferably the catalyst composition comprises for example 0.01 — 10 wt%, for example 0.02 — 5.0 wt % alkali metal and/or alkaline earth metal $M_A$ with respect to the total $M_N/M_A$/Ga-zeolite. It is understood that by wt % of alkali metal and/or alkaline earth metal $M_A$ is meant the sum of the total amount of alkali metal and of the total amount of alkaline earth metal present in the catalyst composition of the invention.

Furthermore, the catalyst composition comprises gallium (Ga). Gallium is present in the catalyst composition in for example at least 0.2 wt %, for example at least 0.3 wt %, for example at least 0.4 wt %, for example at least 0.5 wt % and/or for example at most 0.75 wt %, for example at most 1.0 wt %, for example at most 1.5 wt %, for example at most 2.0 wt % Ga with respect to the total $M_N/M_A$/Ga-zeolite. Preferably the catalyst composition comprises 0.2 to 2 wt % Ga with respect to the total $M_N/M_A$/Ga-zeolite. Most preferably, the catalyst composition comprises 0.5 to 1.5 wt-% Ga with respect to the total $M_N/M_A$/Ga-zeolite, since this further improves conversion and BTX selectivity.

The gallium element and the noble metal contained in the catalyst composition according to the invention may be present in the zeolite structure as a framework or non-framework element as a counterion in the zeolite, or on its surface, e.g. in the form of metal oxides, or be present in a combination of these forms.

The location of gallium in the zeolite structure is largely determined by the method by which gallium is introduced to the zeolite. $Ga_2O_3$ modification of ZSM-5 zeolite catalyst composition using the impregnation method according to the invention leads to the formation of a dispersed oxide phase deposited on the surface. The individual gallium oxide individual active centres participate in the formation of alkene/carbocation intermediates during the reaction process according to the invention. Insertion of gallium by ion exchange methods leads to the formation of the catalyst composition having increased gallium dispersion (exchangeable sites). Preferably the gallium is finely dispersed and substantially present in the exchangeable sites of the zeolite (MFI type).

*In situ* hydrothermal synthesis of the Ga-zeolite catalyst is expected to lead to significant amounts of Ga in the zeolite (MFI) framework along with finely dispersed gallium oxide on the surface and also on the exchangeable sites of the zeolite.

In a special embodiment the invention relates to a composition of the invention wherein the noble metal is Pt and the Pt is present in 0.01-10 wt% with respect to the total $M_N/M_A$/Ga-zeolite, wherein the alkali metal and/or alkaline earth metal is Cs and the Cs is present in 0.01- 10 wt % with respect to the total $M_N/M_A$/Ga-zeolite, wherein the zeolite is ZSM-5 and wherein the ZSM-5 is modified with Ga or was prepared *in situ* using Ga and ZSM-5 precursors, wherein the Ga is present in 0.5-2 wt % with respect to the total $M_N/M_A$/Ga-zeolite and wherein the Ga is finely dispersed on Ga impregnated/exchanged ZSM-5 and/or distributed in the MFI framework.

The catalyst composition may comprise further components such as diluents or binders or other support materials. Preferably these further components do not negatively affect the catalytic performance of the catalyst composition of the invention. Such components are known to the skilled person.

For example, the catalyst composition of the invention may further comprise a non-acidic inert diluent. Preferably the non-acidic inert diluent is quartz (crystalline silicon oxide).

For example, the catalyst composition of the invention may further comprise a binder. Examples of suitable support or binder materials include metal oxides, mixed metal oxides, clays, metal carbides and metal oxide hydroxides. The metal oxide or the mixed metal oxides may be chosen from the group of metal oxides comprising for example, oxides of magnesium, aluminium, titanium, zirconium and silicon. The clay may be, but is not limited to, kaolin, montmorillonite or betonite. Metal carbides suitable for use in the composition of the invention are, for example, molybdenum carbide and silicon carbide. The metal oxide hydroxide may be feroxyhyte, goethite, or more preferably boehmite

The binder may be present in the composition according to the invention in for example at least 5 wt %, for example at least 10 wt%, for example at least 20 wt %, for example at least 30 wt %, for example at least 40 wt %, for example at least 50% and/or for example at most 5 wt %, for example at most 10 wt%, for example at most 20 wt %, for example at most 30 wt %, for example at most 40 wt %, for example at most 50 wt% with respect to the total catalyst composition.

If the zeolite catalyst composition is to contain a binder, such catalyst composition can be obtained, for example, by mixing the modified zeolite and a binder in a liquid or solid mixture, and forming the mixture into shapes, like pellets or tablets, applying methods known to the skilled person.

The catalyst composition used in the present process can be prepared by suitable methods of preparing and modifying zeolites as well known to the skilled person; including for example impregnation, calcination, steam and/or other thermal treatment steps. Such methods are disclosed for instance in documents US 7186872B2; US4822939 and US4180689 hereby incorporated by reference.

Therefore, in a further aspect, the invention relates to a process for preparing the catalyst composition of the invention comprising the steps of:

- preparing the Ga-zeolite by hydrothermal synthesis and/or depositing Ga on the zeolite to provide Ga-zeolite
- depositing alkali metal and/or alkaline earth metal on the Ga-zeolite to provide $M_A$/Ga-zeolite
- depositing noble metal on the $M_A$/Ga-zeolite to provide $M_N/M_A$/Ga-zeolite

It is also possible to combine the step of depositing the alkali metal and/or the alkaline earth metal on the Ga-zeolite and the step of preparing the Ga-zeolite by hydrothermal synthesis by adding salt and/or hydroxides of the alkali metal and/or alkaline earth metal during the hydrothermal synthesis of Ga-zeolite.

Hydrothermal synthesis is a well-known method to the person skilled in the art.

Hydrothermal synthesis employs a dissolution/recrystallization mechanism. The reaction medium along with zeolite and precursors for $M_N$, $M_A$ and Ga also contains structuring agents which are incorporated in the microporous space of the zeolite network during crystallization, thus controlling the construction of the network and assisting to stabilize the structure through the interactions with the zeolite components.

The Ga may (also) be deposited onto the zeolite by ion-exchange and/or impregnation with a solution comprising a soluble salt of gallium (Ga), preferably, an aqueous solution of a soluble salt of gallium, preferably gallium(III) nitrate.

Preferably the alkali metal and/or alkaline earth metal is deposited on the Ga-zeolite by impregnation, soaking, ion-exchange methods and/or during hydrothermal synthesis using a soluble salt and/or a soluble hydroxide of the alkali metal and/or alkaline earth metal. Preferred salts of the alkali metal and/or alkaline earth metal comprise cations of the alkali metal and/or alkaline earth metals chosen from the group of sodium (Na), potassium (K), rubidium (Rb), cesium (Cs), magnesium (Mg), calcium (Ca), strontium  (Sr) and barium (Ba). Preferably the salt of the alkali metal and/or alkaline earth metal is cesium.
Examples of soluble hydroxides of the alkali metal and/or the alkaline earth metal include but are not limited to hydroxides of sodium (Na), potassium (K), rubidium (Rb), cesium (Cs), magnesium (Mg), calcium (Ca), strontium (Sr), barium (Ba) and mixtures thereof.

Preferably the noble metal in the above defined $M_N/M_A$/Ga-zeolite is prepared by ion-exchange and/or impregnation methods, for example (incipient) wetness impregnation with a solution comprising a soluble salt a noble metal, preferably, an aqueous solution of a soluble salt of a noble metal. Preferably, the soluble salt of a noble metal metal used to prepare the solution is selected from the group consisting of tetraamine metal chloride salts, wherein the metal is chosen from the group of platinum (Pt), palladium (Pd), iridium (Ir), rhodium (Rh) and ruthenium (Ru). Preferably the noble metal is platinum (Pt).

For incipient wetness or wetness impregnation, as used in the present invention, a minimum amount of solvent, preferably water, is used to dissolve the metal salt which as an aqueous solution of the salt is sufficient to soak the catalyst and prepare a dry thick paste.

The process for the preparation of the catalyst composition may also contain the step of mixing the $M_N/M_A$/Ga-zeolite with a non-acidic inert diluent in a ratio of for example 1:1 to 3:1, for example of about 2:1.

In a further aspect, the invention relates to a process for the production of aromatic hydrocarbons comprising the step of contacting a feedstream comprising an alkane selected from the group of alkanes having from 3 to 12 carbon atoms per molecule and any mixtures of alkanes having from 3 to 12 carbon atoms per molecule with the catalyst composition according to the invention to form aromatic hydrocarbons and wherein the feedstream comprises hydrogen in a molar ratio of hydrogen to alkane in the range from about 6:1 to 0:1.

The number of carbon atoms present in the alkane may vary, for example from 3 to 8 carbon atoms per molecule or for example from 6 to 8 carbon atoms per molecule, or for example from 6 to 12 carbon atoms per molecule may be present. Preferably the alkanes used have from 3 to 8 carbon atoms per molecule. A mixture of alkanes having  6 to 12 carbon atoms per molecule is known as petroleum naphtha, whereas a mixture of alkanes having 6 to 8 carbon atoms is known as light naphtha.

In a special embodiment, the alkanes having 3 to 12 carbon atoms per molecule may be chosen from the group of alkanes having 6 carbon atoms per molecule, alkanes having 7 carbon atoms per molecule and having 8 carbon atoms per molecule and any mixtures thereof.

The alkane may be used in its pure form, but may also be present in a feedstream of a mixture of alkanes or in a feedstream of alkane (also referred to herein as alkane feedstream) with an inert gas, such as $N_2$. Preferably, the alkane is present in a feedstream that predominantly comprises one alkane species. For the avoidance of doubt in this application the term "alkane group" and "alkane species" are used interchangeably.

Accordingly, it is preferred that the alkane comprised in the feedstream consists of at least 75 mol % of only one alkane species, more preferably of at least 85 mol % of only one alkane species, even more preferably of at least 90 mol % of only one alkane species, particularly preferably of at least 95 mol % of only one alkane species and most preferably of at least 98 mol % of only one alkane species

Preferably, the total amount of alkane in the feedstream is at least 98 wt%, preferably at least 99 wt%, for example at least 99.5 wt%, for example at least 99.7 wt%, for example 99.9 wt% based on the total feedstream. Small amounts of olefins (for example from 0.1 to 0.5wt% based on the total feedstream) and trace amounts of sulphur (for example 10-100ppm based on the total feedstream) may be present in the feedstream.

The feedstream may also comprise hydrogen. For example, the molar ratio of hydrogen to alkane in the feedstream may be in the range from about 6:1 to 0:1.

The feedstream may also comprise an inert gas diluent. The inert gas diluent may be chosen from the group of helium, nitrogen, and mixtures thereof. For example, in case hydrogen is present in the feedstream, the molar ratio of inert gas diluent to hydrogen may be in the range from about 0.5:1 to about 3:1.

The terms "aromatic hydrocarbon" is very well known in the art. Accordingly, the term "aromatic hydrocarbon" relates to cyclically conjugated hydrocarbon with a stability (due to delocalization) that is significantly greater than that of a hypothetical localized structure (e.g. Kekulé structure). The most common method for determining aromaticity of a given hydrocarbon is the observation of diatropicity in the $^1$H NMR spectrum, for example the presence of chemical shifts in the range of from 7.2 to 7.3 ppm for benzene ring protons. The aromatic hydrocarbons produced in the process of the present invention are preferably benzene, toluene and xylenes, more preferably benzene.

The mixture of aromatic hydrocarbons produced, therefore, may comprise for example at least 70 mol %, for example at least 80 mol%, for example at least 90 mol %, for example at least 95 mol % , for example at least 96mol%, for example at least 97mol% and/or for example at most 100 mol % benzene with respect to the total amount of the aromatic hydrocarbon produced. For example, the aromatic hydrocarbon produced is for example from 70 to 100 mol %, for example from 80 to 100 mol %, for example from 90 to 100 mol % benzene with respect to the total amount of the aromatic hydrocarbon, preferably the total amount of benzene, toluene and xylene produced.

The process of the present invention is performed at conditions suitable for high conversion of an alkane to an aromatic hydrocarbon, such conditions are known by the person skilled in the art; see e.g. O'Connor, Aromatization of Light Alkanes. Handbook of Heterogeneous Catalysis Wiley-VCH 2008, pages 3123—3133. Optimal conditions can easily be determined by the person skilled in the art using routine experimentation.

The process for the production of aromatic hydrocarbons according to the invention may be performed across a temperature range of, for example 275 to 650 C. A higher temperature generally enhances conversion to aromatic hydrocarbons; therefore, the temperature is preferably at least 400 °C. Very high temperatures may induce side-reactions or promote deactivation of the catalyst composition and so the temperature is preferably at most 650 °C. The temperature is preferably at least 300 °C, for example at least 350 °C, for example at least 400 °C and/or preferably for example at most 450 °C, for example at most 500 °C, for example at most 550 °C, for example at most 600 °C. For example the temperature of the process according to the invention ranges from 350 °C to 600 °C.

Suitable pressures for the process for the production of aromatic hydrocarbons according to the invention are for example from about atmospheric pressure (around 0.1MPa) to 3 MPa, preferably pressure is below about 2.5, 2.0, 1.5, 1.0, 0.5 or even below 0.3 MPa.

The flow rate at which the feedstream comprising alkanes having 3 to 12 carbon atoms per molecule is fed to the reactor may vary widely, but is preferably such that a weight hourly space velocity (WHSV) results of about 0.1 — 100 h$^{-1}$, more preferably WHSV is about 0.5 -50 h$^{-1}$, or 1 — 20 h$^{-1}$ or most preferably 2.0 -4.0 h$^{-1}$. The WHSV may be preferably at least 0.1 h$^{-1}$, for example at least 10 h$^{-1}$, for example at least 20 h$^{-1}$, for example at least 30 h$^{-1}$and/or for example at most 1 h$^{-1}$, for example at most 10 h$^{-1}$, for example at most 20 h$^{-1}$, for example at most 30 h$^{-1}$, for example at most 40 h$^{-1}$, for example at most 50 h$^{-1}$. WHSV is the ratio of the rate at which the feedstream is fed to the reactor (in weight or mass per hour) divided by the weight of catalyst composition in a reactor; and is thus inversely related to contact time.

By contact time is meant the period of time during which the alkane feedstream is in contact with the catalyst composition.

The WHSV indicates that there is a certain rate at which the feedstream is fed to the reactor. The total length of time in which the feedstream is fed to the reactor is known as the "Time-on-Stream (TOS)." The TOS may be for example at least 50 hours, for example at least 75 hours, for example at least 100 hours, for example at least 150 hours and/or for example at most 50 hours, for example at most 75 hours, for example at most 100 hours, for example at most 150 hours, for example at most 200 hours. For example the TOS for a catalyst composition according to the invention during which time the catalyst composition maintains its activity in terms of a high conversion and high selectivity for benzene, ranges from for example 50 to 200 hours, for example from 100 to 150 hours.

The step of contacting the alkane with the zeolite catalyst composition can be performed in any suitable reactor, as known to a skilled man, for example in a fixed bed, a fluidized bed, or any other circulating or moving bed reactor.

In yet another aspect the invention relates to the use of the catalyst composition according to the invention in the production of aromatic hydrocarbons.

Although the invention has been described in detail for purposes of illustration, it is understood that such detail is solely for that purpose and variations can be made therein by those skilled in the art without departing from the spirit and scope of the invention as defined in the claims.

It is further noted that the invention relates to all possible combinations of features described herein, preferred in particular are those combinations of features that are present in the claims.

It is further noted that the term 'comprising' does not exclude the presence of other elements. However, it is also to be understood that a description on a product comprising certain components also discloses a product consisting of these components. Similarly, it is also to be understood that a description on a process comprising certain steps also discloses a process consisting of these steps.

The invention is now elucidated by way of the following examples, without however being limited thereto.

Examples

Example 1. Preparation of Ga-ZSM-5 zeolite

Solution A was prepared by dissolving 0.52 g of sodium aluminate and 2.387 g sodium hydroxide in 15.0 ml demineralised water. To a suspension containing 45.0 g ludox (40% in water) in 45.0 ml demineralised water, solution A was added slowly under vigorous stirring using a dropping funnel to prepare the synthesis gel. Solution B was prepared by dissolving 0.36 g gallium nitrate in 5 ml demineralised (DM) water and solution C was made by diluting 31.92 g tetrapropylammonium hydroxide (TPAOH) 1.0 M solution with 95 ml demineralised water. Solution B and solution C were added to the synthesis gel sequentially under stirring and the mixture was allowed to stir for additional 30 minutes. The final mixture was loaded into a 300 ml Parr autoclave reactor and heated at 160 °C under stirred conditions for 24 hours for the first phase of crystallization. Subsequently, the Parr reactor was cooled to 30-40 °C and the mixture was transferred to a polypropylene (PP) beaker and the pH of the mixture was adjusted to about 9 while stirring using acetic acid. The mixture was allowed to stir for an additional 30 minutes and then transferred to the Parr autoclave for a second phase of crystallization at 160 °C with stirring for another 24 hours. After two phases of crystallization, the solids obtained were filtered, washed with DM water, dried overnight at 110 °C and calcined at 550 °C for 6 hours in dry air.

Example 2. Preparation of Cs/Ga-ZSM-5 zeolite

9.84 g cesium nitrate was dissolved in 100 ml DM water in a PP beaker. 4.0 g of dry Ga-ZSM-5 of example 1 was added slowly under magnetic stirring at room temperature (RT) and stirring was continued for 10 minutes. After the first exchange, the solids were filtered under vacuum and the wet cake was mixed with same amount of cesium nitrate aqueous solution for a second phase exchange. After the second exchange, the solids were filtered under vacuum, dried at 110 °C for overnight and calcined at 280 °C for 2 hours in air.

Example 3. Preparation of PUCs/Ga-ZSM-5 zeolite

0.0378 g Tetra ammine platinum (II) chloride hydrate was dissolved in 2 ml DM water in a PP beaker. 2.2 g of dry Cs/Ga-ZSM-5 of example 2 was taken in a silica bowl and slowly tetra amine platinum (II) chloride solution was added to the Cs/Ga-ZSM 5 and mixed with a spatula to make a thick paste. The obtained material was dried overnight at 110 °C and subsequently calcined at 280 °C for 3 hours in air.

Example 4. Preparation of catalyst particles

A number of catalyst compositions comprising different zeolites and binder supports were prepared in particle form by mixing the zeolite and the binder support thoroughly in a 2:1 ratio. The mixture was pressed at 10 ton pressure to make pellets. The pressed catalyst compositions were crushed and sieved. The fraction containing particles from 0.25 to 0.5 mm and the fraction containing particles from 0.5 to 1.00 mm particles were selected for further use. The particles of the active zeolite component, and binder were also prepared separately after which the two components (in particle forms) were mixed in a 2:1 ratio (wt/wt) to prepare the final catalyst composition and perform the catalytic testing. When quartz was used as diluent, the quartz tubes were crushed and sieved and then quartz particles were mixed with catalyst particles for catalytic screening.

Example 5. Catalyst testing

Two grams catalyst particles (particle size 0.25-0.5 mm) were loaded in a down flow fixed bed micro catalytic reactor and pre-treated in the following way:

Step 1: Exposed for 1 h to moisture-free air flow of 25 ml/min at 580 °C and nitrogen was passed until the temperature came down to 525°C

Step 2: Exposed for 30 min to 200 ml/min hydrogen flow at 525 °C.

After the pre-treatment, *n*-hexane was fed to the bed. The temperature of the catalyst bed before the start of the *n*-hexane flow was 525 °C. The Weight Hourly Space Velocity (WHSV) was 2.0 h$^{-1}$.

Unconverted *n*-hexane and formed products were analysed by an on-line Gas Chromatograph, separation column Petrocol DH 50.2, using a Flame Ionization Detector.

After the reaction, the catalyst was regenerated in the following way:

Step 1: Exposed for 4 h in nitrogen gas (270 ml/min) with 2 vol. % of moisture-free air at 540 °C;

Step 2: The reactor was cooled to 150 °C, start passing steam with nitrogen for 30 min (N$_2$ flow = 50 ml/min, Water flow = 0.0021 ml/min). This step is optional and was carried out once after five to ten cycles

Step 3: Increased the reactor temperature up to 525 °C with nitrogen gas (76 ml/min)

Step 4: Exposed for 30 min to 200 ml/min hydrogen flow at 525 °C.

After the regeneration of the catalyst, *n*-hexane was fed to the bed (WHSV = 2.0 h$^{-1}$) and the aromatization reaction was continued.

The provided values were calculated as follows:

Conversion:

An indication of the activity of the catalyst was determined by the extent of conversion of the *n*-hexane. The basic equation used was:

$$\text{Conversion mol\%} = \text{Moles of } n\text{-hexane }_{in} - \text{moles of } n\text{-hexane }_{out}/\text{moles of } n\text{-hexane }_{in} * 100/1$$

BTX selectivity

The BTX selectivity is the mol% BTX produced based on the total mol of *n*-hexane converted.

Benzene selectivity

The selectivity of benzene is the mol% of benzene based on the total mol of *n*-hexane converted.

Results

Table 1 provides the catalytic performance (conversion, BTX, and benzene selectivity) and catalyst stability for *n*-hexane aromatization (Reaction temperature = 525 °C, Pressure = 1 atmosphere, WHSV = 2.0 h$^{-1}$) for three cycles. Reactions were conducted for 2 hours on each cycle and 1.5 hours data is presented. The catalyst was regenerated after each reaction cycle.

Table 2 provides the catalytic performance (conversion, BTX and benzene selectivity) studies against time-on-stream for *n*-hexane aromatization (Reaction temperature = 525 °C, Pressure = 1 atmosphere, WHSV = 2.0 h$^{-1}$).

For both tables, the catalyst used was 0.9wt%Pt/5.7wt%Cs/1wt%Ga-HZSM-5(55) (wt% are given based on the total Pt/Cs/Ga-HZSM-5(55)); Quartz was taken as binder. Active component to binder ratio was considered as 2:1 (wt/wt) for the final catalysts composition.

As can be seen from Table 1, catalysts of the invention show a reproducible conversion, BTX and benzene selectivity for aromatics when aromatics are prepared from light naphtha, in this case from *n*-hexane. Moreover, catalysts of the invention show a high selectivity for benzene (see entry 1-3 in table 1).

As can be seen from Table 2, catalyst of the invention showed a high selectivity for aromatics when aromatics are prepared from light naphtha, in this case *n*-hexane. Moreover, catalyst of the invention showed a high selectivity for benzene and/or a high yield for benzene (see entry 1-3 in table 2) even after 96 hours time-on-stream. This shows that catalysts of the invention maintain their activity over long periods of time.

**Table 1:** Comparison of catalytic performance and catalyst stability studies for n-hexane aromatization reaction using catalyst composition comprising 0.9%Pt/5.7%Cs/1%Ga-HZSM-5(55) + quartz (2:1) as principal components

| No. of Cycles | Reaction Time / h | $n$-Hexane Conversion / % | BTX Selectivity / % | Benzene Selectivity / % |
|---|---|---|---|---|
| Cycle 1 | 1.5 | 100 | 72.6 | 72.4 |
| Cycle 2 | 1.5 | 100 | 73.6 | 73.3 |
| Cycle 3 | 1.5 | 100 | 75.8 | 75.4 |

**Table 2:** Catalytic performance studies against time-on-stream (TOS)studies for n-hexane aromatization reaction using catalyst composition comprising 0.9%Pt/5.7%Cs/1%Ga-HZSM-5(55) + quartz (2:1) as principal components

| Time-On-Stream (TOS) / h | $n$-Hexane Conversion / % | BTX Selectivity / % | Benzene Selectivity / % |
|---|---|---|---|
| 2 | 99.8 | 78.3 | 77.9 |
| 24 | 99.6 | 74.7 | 74.4 |
| 46 | 97.3 | 75.2 | 74.8 |
| 70 | 97.4 | 74.7 | 74.4 |
| 96 | 86.8 | 74.8 | 74.4 |

**Claims**

1. Catalyst composition suitable for conversion of alkanes having 3 to 12 carbon atoms per molecule to aromatic hydrocarbons, wherein the catalyst composition comprises $M_N$/$M_A$/Ga-zeolite, wherein $M_N$ stands for one or more noble metals and $M_A$ stands for one or more alkali metals and/or alkaline earth metals, and wherein $M_N$/$M_A$/Ga-zeolite is a zeolite comprising 0.01 -10 wt % of $M_N$ with respect to the total $M_N$/$M_A$/Ga-zeolite, 0.01 — 10 wt % of $M_A$ with respect to the total $M_N$/$M_A$/Ga-zeolite and 0.01 — 10 wt % Ga with respect to the total $M_N$/$M_A$/Ga-zeolite.

2. Catalyst composition according to claim 1 wherein $M_N$ is Pt.

3. Catalyst composition according to claim 1 or claim 2, wherein $M_A$ is Cs.

4. Catalyst composition according to claims 1 - 3, wherein the composition further comprises a non-acidic inert diluent.

5. Catalyst composition according to claim 4, wherein the non-acidic inert diluent is quartz

6. Catalyst composition according to any one of claims 1-5, wherein the composition further comprises a binder.

7. Catalyst composition according to claim 6, wherein the binder is selected from the group of metal oxides, mixed metal oxides, clays, metal carbides, metal nitrides and metal oxide hydroxides.

8. Catalyst composition according to any one of claims 1-7, wherein the zeolite is an MFI zeolite, preferably ZSM-5 zeolite.

9. Process for preparing the catalyst composition of any one of claims 1-8 comprising the steps of:

   — preparing the Ga-zeolite by hydrothermal synthesis and/or depositing Ga on the zeolite to provide Ga-zeolite
   — depositing alkali metal and/or alkaline earth metal on the Ga-zeolite to provide $M_A$/Ga-zeolite
   — depositing noble metal on the $M_A$/Ga-zeolite to provide $M_N$/$M_A$/Ga-zeolite

10. Process according to any one of claims 1-9, wherein the alkali metal and/or alkaline earth metal is deposited on the Ga-zeolite by impregnation, soaking, ion-exchange methods and/or during hydrothermal synthesis using a soluble salt and/or a soluble hydroxide of the alkali metal and/or alkaline earth metal.

11. Process according to any one of claims 1-10, wherein the noble metal is deposited on $M_A$/Ga-zeolite by impregnation with a solution comprising a soluble salt of the noble metal.

12. Process for the production of aromatic hydrocarbons comprising the step of contacting a feedstream comprising an alkane selected from the group of alkanes having from 3 to 12 carbon atoms per molecule and any mixtures of alkanes having from 3 to 12 carbon atoms per molecule with the catalyst composition of any one of claims 1-8 to form aromatic hydrocarbons and wherein the feedstream comprises hydrogen in a molar ratio of hydrogen to alkane in the range from about 6:1 to 0:1.

13. Process according to claim 12 wherein the alkanes having 3 to 12 carbon atoms per molecule comprise an inert gas diluent.

14. Process according to claim 12 or claim 13 wherein the aromatic hydrocarbons comprise benzene, toluene and xylenes, preferably benzene.

15. Use of the catalyst composition of any one of claims 1-8 in the production of aromatic hydrocarbons.

Europäisches
Patentamt

European
Patent Office

Office européen
des brevets

# EUROPEAN SEARCH REPORT

Application Number

EP 12 00 5628

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 62 081329 A (INUI SATOYUKI) 14 April 1987 (1987-04-14) * the whole document * ----- | 1-15 | INV. B01J29/87 B01J29/44 C10G35/06 C10G35/095 C07C2/76 C07C15/02 |
| X | US 2005/197515 A1 (JUTTU GOPALAKRISHNAN G [US] ET AL) 8 September 2005 (2005-09-08) * abstract * * paragraphs [0002], [0021] - [0024], [0029] - [0030], [0034], [0036] * * example 1 * ----- | 1,2,4-15 | |
| A | US 5 456 822 A (MARCILLY CHRISTIAN [FR] ET AL) 10 October 1995 (1995-10-10) * the whole document * ----- | 1-15 | |
| A | TOMOYUKI INUI ET AL: "Selective aromatization of light paraffins on platinum-ion-exchanged gallium-silicate bifunctional catalysts", INDUSTRIAL & ENGINEERING CHEMISTRY RESEARCH, vol. 26, no. 4, 1 April 1987 (1987-04-01), pages 647-652, XP055052038, ISSN: 0888-5885, DOI: 10.1021/ie00064a002 * the whole document * ----- | 1-15 | |

TECHNICAL FIELDS SEARCHED (IPC)

B01J
C10G
C07C

The present search report has been drawn up for all claims

| Place of search | Date of completion of the search | Examiner |
|---|---|---|
| Munich | 7 February 2013 | Jourdan, Angelika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**                    EP 12 00 5628

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2013

| Patent document cited in search report | | Publication date | Patent family member(s) | | Publication date |
|---|---|---|---|---|---|
| JP 62081329 | A | 14-04-1987 | JP | 7080795 B | 30-08-1995 |
| | | | JP | 62081329 A | 14-04-1987 |
| US 2005197515 | A1 | 08-09-2005 | CN | 1938245 A | 28-03-2007 |
| | | | EP | 1725510 A1 | 29-11-2006 |
| | | | RU | 2367643 C2 | 20-09-2009 |
| | | | US | 2005197515 A1 | 08-09-2005 |
| | | | WO | 2005085157 A1 | 15-09-2005 |
| US 5456822 | A | 10-10-1995 | AU | 1397792 A | 08-10-1992 |
| | | | EP | 0507656 A1 | 07-10-1992 |
| | | | FR | 2674769 A1 | 09-10-1992 |
| | | | US | 5281566 A | 25-01-1994 |
| | | | US | 5456822 A | 10-10-1995 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1296861
- EP 0283212 A
- US 7164052 B
- US 5006497 A
- WO 2008080517 A
- WO 2005085157 A1
- US 7186872 B2
- US 4822939 A
- US 4180689 A

**Non-patent literature cited in the description**

- Molecular Sieves in Kirk-Othmer Encyclopedia of Chemical Technology. Atlas of Zeolite Framework Type. Elsevier, 2001, vol. 16, 811-853
- Post-synthesis Modification I; Molecular Sieves. 2002, vol. 3, 204-255
- **O'CONNOR.** Aromatization of Light Alkanes. Handbook of Heterogeneous Catalysis. Wiley-VCH, 2008, 3123-3133